(19) Europäisches Patentamt

European Patent Office

Office européen des brevets

(11) Publication number: **0 418 979 A2**

## (12) EUROPEAN PATENT APPLICATION

(21) Application number: 90202485.0

(22) Date of filing: 18.09.90

(51) Int. Cl.5: **C08H 1/06**, //A61L31/00

(30) Priority: 21.09.89 IT 6778389
28.09.89 IT 6780889

(43) Date of publication of application:
**27.03.91 Bulletin 91/13**

(84) Designated Contracting States:
**CH DE ES FR GB LI NL**

(71) Applicant: **Zocchi, Michele, Dr.**
**Strada privata Del Milus 3**
**I-10024 Moncalieri, Torino(IT)**

(72) Inventor: **Zocchi, Michele, Dr.**
**Strada privata Del Milus 3**
**I-10024 Moncalieri, Torino(IT)**

(74) Representative: **Buchan, Ian Alexander et al**
**Fitzpatricks 4 West Regent Street**
**Glasgow G2 1RS(GB)**

(54) Method and apparatus for producing human autologous collagen.

(57) This invention provides a method and the apparatus for producing human autologous collagen from the human fat tissues and specifically for a method of disintegrating fat cells using ultrasonic energy and separating fats, blood, water and serum from the collagen, all of which can be accomplished without removing material from the harvesting syringe. The purpose of the invention is to produce autologous collagen which is useful in several cosmetic procedures used to treat all the problems caused by ageing. In accordance with the procedure taught in the invention after fat cells have been removed from the subject in a harvesting syringe, first the material in the syringe is subjected to an ultrasonic energy source of a range between 20 kHz and 3mHz which destroys the fat cells by rupturing them which allows the oil in the fat cells to be separated from the residual cell walls, after which the harvesting syringe is placed in a specially designed centrifuge allowing for the blood, serum, fat cell oil and collagen to be completely separated. In the fat cell disintegration step, when applying ultrasonic energy, two alternative devices may be employed with the harvesting syringe containing the material. In one, the syringes are placed in a container filled with a cold liquid and subjected to ultrasonic energy. The second, preferred method utilizes a high intensity ultrasonic processor that includes a probe that can be placed in the harvesting syringe that is mounted individually in a metal support filled with a cold liquid. In the separation stage, a multibodied centrifuge is provided which holds up to four harvesting syringes safely while subjected to centrifugal action to permit the separation of the materials as described herein. With the use of the present invention human autologous collagen can be produced in minutes without removal from the harvesting syringe for use in the operation at the time or for frozen storage in an individual patient collagen bank.

FIG.2

# METHOD AND APPARATUS FOR PRODUCING HUMAN AUTOLOGOUS COLLAGEN

## BACKGROUND OF THE INVENTION

### 1. Field of the Invention

This invention relates to a method and apparatus for producing human autologous collagen from the human fat tissues, and specifically to an improved method and apparatus for producing collagen without removing the treated tissues from the harvesting syringes used for the harvesting allowing in this way to complete the whole procedure in a sealed sterile cycle.

### 2. Description of the Prior Art

Fat transplantation, better known as lipofilling is known to be one of the most efficient cosmetic procedures to treat the problems caused by ageing. The surgical process involves the harvesting of autologous fat tissue and the transfer of the harvested fat tissue to the facial or body areas requiring correction after the tissue is separated from water, saline, blood serum and physiological and aesthetic solutions injected before the surgery. In using this surgical procedure, one of the choices (implant or graft) is to inject just the autologous collagen contained in the cell wall of adipocytes. The problem in using this approach has been in obtaining the collagen necessary to complete the surgical process. In this case, it is an implant and not a graft, and thus the permanence of the material in the injected areas is assured not by a cellular survival but by its progressive integration in the conjunctival structures of the implantation zone. It is just for the experienced use of this last technique, sometimes combined with the first one, that the author has created a new method assuring the achievement of a dense and homogenous product and of the excellent quality, with a complete utilization of the harvested fat tissue. Collagen is an insoluble fibrous protein that occurs in vertebrates as the chief constituent of the fibrils of connective tissue such as skin. To date one process for obtaining collagen was very time consuming and inefficient which involved mixing harvested tissues with distilled water which by its hypotonic capacity increased the volume of the fat cells until they were partially ruptured and destroyed. Another alternative has been to use bovine collagen, a commercial product obtained by treatment of cow's skin. This is a very expensive procedure that cannot be used with all the patients due to the severe allergical reactions to this product.

The present invention overcomes the problem of producing autologous collagen without removing it from the harvesting syringes by the application of ultrasonic energy which acts to destroy the fat cells allowing the mixture to be separated by centrifuge with a highly dense purified collagen resulting from the centrifugal action on the mixture. The invention also embodies an improved centrifuge for use in the separation process.

## SUMMARY OF THE INVENTION

A process and system for producing autologous collagen comprising the harvesting of fat tissue from the patient with the use of aspirating syringes, subjecting the syringes containing the harvested material to ultrasonic energy until the thorough and complete destruction of the fat cells is accomplished, and separating the resultant material in the syringe in a centrifuge which allows the collagen to be separated out from the remaining mixture for resultant injection. In detail it is possible distinguish in the syringes, from below to high, three different layers;

1) in the lower part the blood, the physiological saline and anaesthetic solution used for the harvesting and the rinsing;

2) in the middle, the auto-collagen formed by all cellular residues, the wall of the burst adipocytes and intercellular substances, very isolated;

3) on the surface, a very important oily layer coming from the lipidic matrix of the burst cells.

It is clear that only the middle layer, formed by the autologous collagen interests us for the reinjection. It is very simple by a needle to remove all parasitic layers and so to obtain in all syringes a pure collagen substance.

It is also interesting to note that the process of centrifugation using the centrifuge invented by applicant and described herein can also be used in the traditional lipofilling (fat tissue transplant). The centrifugal force does not cause any structural change on the adypocites and does not affect their capacity of surviving. On the contrary, it considerably reduces times of their manipulation and rinsing, thereby making it possible to regraft them very quickly in the areas to be improved, such that the cells to begin again immediately vascular and metabolic exchanges.

In one embodiment of the method of the invention, with respect to the application of ultrasonic energy to be harvested fat tissue, the syringe containing the harvested fat tissue receives a sterilized ultrasonic probe which exposes the harvested material in the syringe to an appropriate ultrasonic frequency such as approximately 20 kHz for a period of time up to 20 to 25 seconds. The source of ultrasound waves changes the eleotrical current from 110/220 V to 50/60 Hz in an electric energy with a highest frequency from 20 kHz to 3 mHz (20,000-3,000,000 cycles/sec.). This energy at high frequency is transmitted later to the piezo-electrics transducers where it is transformed in mechanical vibrations.

These vibrations are intensified by a resonance chamber by synergy within different transducers (1 or more) and then transmitted to the contained liquid, so creating some waves of pressure.

This operation causes the formation of millions of micro-blisters which increase during the pressure drop wave and implosent violenty during the positive pressure wave. It is exactly this physical phenomenon, better known with the technical term of cavitation, which produces this great action of cellular fragmentation. During cavitation free radicals are formed which, if they are allowed to accumulate, can greatly affect the biological integrity of the sample. Although during short periods of processing their formation is not normally considered a problem. To avoid this side effect the probe is immersed in the the harvested tissues contained by the syringe through the top of the syringe after removing the piston and held in a vertical position in a stainless steel container filled with cold sterile saline mixed with alcohol to allow lower temperatures.

Another alternative is to saturate the solution with hydrogen or carbon dioxide that often eliminates free radical formation. In this case a small pellet of dry ice dropped in the solution will often resolve the problem. It is important to avoid the overheating of the tissues (not to exceed 60 C°) due to the energy provided by the ultrasonic probe.

During such a process, the coolant solution in which the syringes are suspended absorbs any excess heat generated by the process, avoiding the possibility of overheating the material processed which might cause unwanted alteration of such material. After the period of exposure to the ultrasonic energy, the tissues come out finely fragmented and homogenized. After the probe has been removed, the syringe is transferred to a centrifugation process which separates the collagen from all the other harvested materials. It is applicant's position that exposure to the proper ultrasonic frequency for the correct amount of time acts to disintegrate the cell walls by implosion allowing the oil in the cells to escape. Because of the action of a centrifuge, the different densities of the harvested materials and ruptured cells permit a clear and defined separation in different layers of collagen and the remaining materials.

In an alternate embodiment, a plurality of syringes containing harvested material may be put into a container filled with a cold sterile saline solution. The walls of the container transmit the action of several piezo electric transducers generating vibrations at a frequency between 20 kHz and 3 Mhz for a period of time of between 15 and 25 minutes. Again, once the ultrasonic vibration energy has destroyed the fat cells completely, the syringes are placed in a centrifuge for separation. The cold saline solution prevents the harvested material from becoming overheated.

When utilizing a probe for ultrasonic energy that is immersed into the harvested material contained by the syringe for the total disintegration of the fat cells, the probe may be based on ultrasonic processors that are currently for sale that have been used previously for emulsification and the like of laboratory samples. One such example is a product known as Vibra Cell that is sold by Sonics and Materials. Inc., Danbury, Connecticut. One other example is a product known as Lipotrit 1 that is designed by the inventor and sold by Bielsan S.r.l. Electro-Medical Equipment, Milan, Italy.

With respect to the centrifuge, applicant's invention also includes an improved centrifuge in which four separate chambers are provided such that each chamber houses a syringe without the needle but with the piston in full extension to ensure the proper spinning action without damage to the syringe itself.

It is an object of this invention to provide an improved method for producing autologous collagen for use in the implantation during liposculpturing surgery of the human body.

It is an object of this invention to provide apparatus for the production of collagen for use in the implantation in liposculpturing surgical procedures of the human body.

It is yet another object of this invention to provide an improved process and equipment that greatly reduces the cost and time required to collect autologous collagen from human fat cells which are used for aesthetic surgery procedures on human beings.

Yet still another object of the invention is to provide a method and apparatus for producing human collagen which is obtained in a closed and sterile environment rapidly and with simplicity by the surgeon himself while performing a lipofilling procedure or which may be frozen and preserved in a sealed and sterile envelope to develop a collagen bank for the patient.

In accordance with these and other objects which will be apparent hereinafter, the instant invention will now be described with particular reference to the accompanying drawings.

## BRIEF DESCRIPTION OF THE DRAWINGS

Figures 1a, 1b, 1c show a schematic diagram of a syringe which houses the harvested material throughout the disintegration and separation stages of the process.

Figure 1d shows a schematic diagram of the apparatus used to ultrasonically disintegrate the fat cells prior to separation.

Figure 2 shows an alternate embodiment in a schematic diagram for providing the ultrasonic disintegration of fat cells prior to separation.

Figure 3 shows a side elevational view of a centrifuge used with the present invention.

Figure 4 shows a top plan view of the centrifuge shown in Figure 3.

## PREFERRED EMBODIMENT OF THE INVENTION

The method in accordance with the preferred embodiment of the invention comprises the steps of harvesting human fat tissue (which includes autologous collagen) from the patient, collecting the fat cells in a syringe, surrounding the syringe with a coolant, subjecting the harvested material while it remains in the sterile environment of the syringe to an ultrasonic energy force that acts to disintegrate and destroy the fat cells by rupturing the cell walls within the syringe, and subjecting the syringe with the material to centrifugation which allows for separation of the harvested tissue into layers being such that the collagen will collect in a predetermined layer.

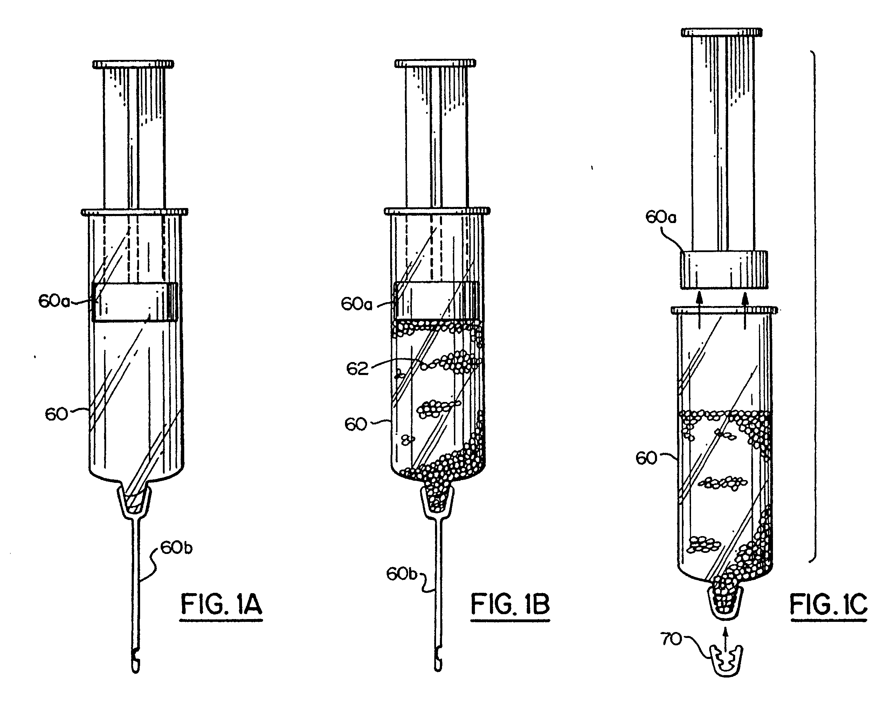

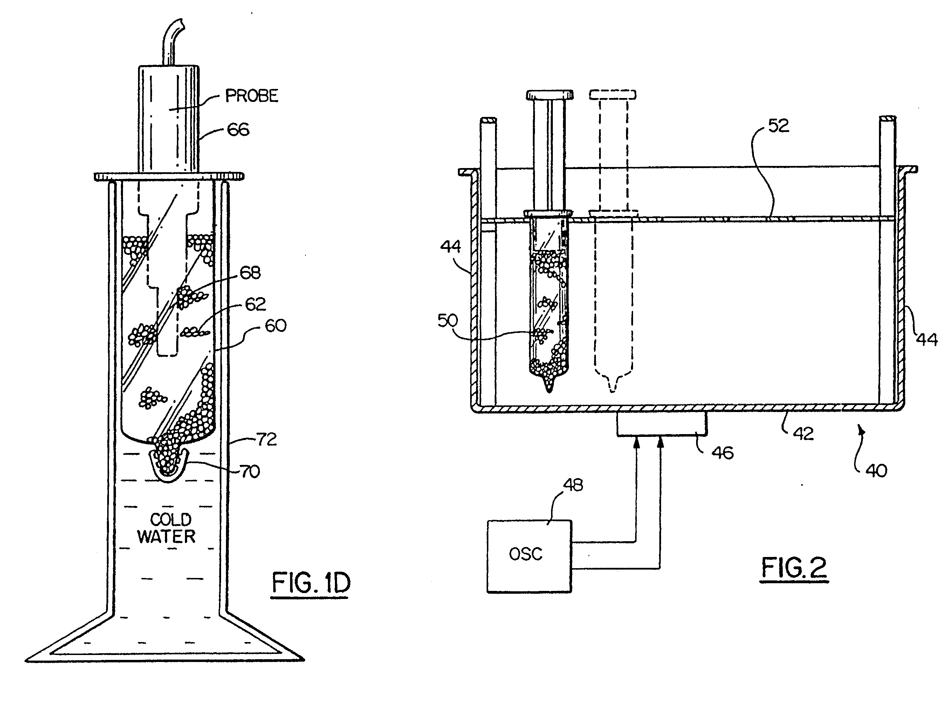

The preferred apparatus for applying ultrasonic energy to the harvested material in the syringe, is shown in Figure 1d. Figures 1a, 1b and 1c show a typical syringe 60 having harvested fat cells 62. Figure 1a shows a conventional syringe 60 used to collect or harvest human fat cells that includes a plunger 60a and needle 60b. Figure 1b shows the syringe 60b with harvested fat cells 62 received from a patient. In order to subject the harvested cells 62 with the ultrasonic energy, the plunger 60a and needle are removed from syringe 60 as shown in Figure 1c. The opening that provides for the needle 60b attachment is covered and sealed by plug 70. In Figure 1d, the syring 60 is placed in a stainless steel support vessel 72 containing cold sterilized liquid (saline and alcohol solution) that surrounds a large portion of the syringe body 60. The cold liquid ensures that the harvested material 62 will not become overheated because of the application of ultrasonic energy transmitted from probe 68 extending from upper body portion 66. The probe 68 extends from ultrasonic wave generator that produces energy at 20 kHz in the harvested material 62 for 2 to 3 minutes for volumes of 10cc to 60 cc or as required. Such an ultrasonic device could be the vibra cell VC50 which is sold by Sonics and Matèrials Inc. of Danbury, Connecticut or the LIPOTRIT 1 designed by the inventor and sold by Bielsan, S.R.1. Electro-Medical Equipment, Milano, Italy. As shown in Figure 1d, such ultrasonic energy device 66 includes a pre-sterilized probe 68 which is disposed in syringe 60 containing harvested cell material 62 with the probe 68 being disposed through the top of the syringe 60. In the preferred embodiment the material 62 in the syringe 60 will be exposed for a period of time of approximately twenty to thirty seconds to the actionof the probe 68 which produces vibrations of a frequency of approximately 20 kHz in the harvested material causing cavitations of high and low pressure wave energy which acts to disintegrate and rupture the fat cell tissues in a very short period of time. Once the total disintegration of the fatty tissue is complete, the probe 68 is removed from the syringe and the syringe then placed in a centrifuge as is discussed below.

Figure 2 shows an alternate embodiment of the invention which also provides ultrasonic energy to a syringe containing harvested material. In Figure 2 the syringe 50 is shown mounted in a rack 52 all of which is mounted in an ultrasonic transmitting container 44 which is attached to a transducer 46 coupled to a source of oscillating energy 48 along the bottom 42 of container 44. The container may also include a suitable cold sterile solution (saline and 60% alcohol) to prevent the harvested material from becoming overheated from the ultrasonic energy (not to exceed 60° c). The harvested material in syringe 50 (the container 44 could hold several syringes) is subjected then to an ultrasonic energy from 20 kHz to 3 mHz for a period of time of approximately fifteen to twenty minutes. Again the fat tissue is disintegrated by the cavitation action created by high and low sonic pressure waves within the material causing cells to rupture and be destroyed. After the process of treating the harvested materials in the syringes with ultrasonic energy is complete, the syringes are then removed to a centrifuge that is described below. The plunger or piston 60a is replaced in each syringe prior to centrifugation.

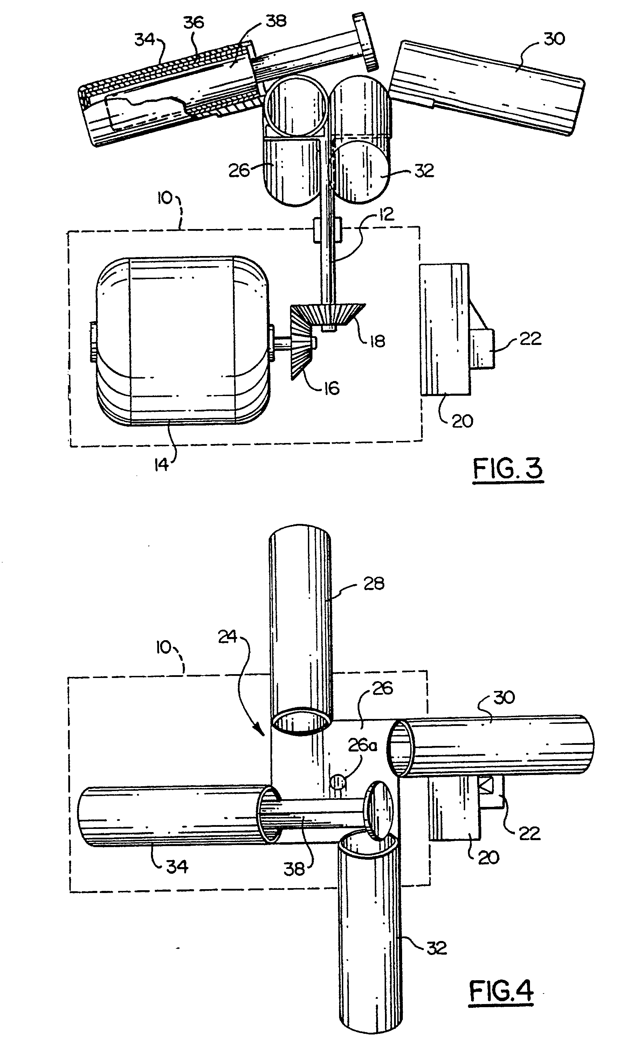

Referring now to Figures 3 and 4, the centrifuge in accordance with the present invention is comprised of base 10 which supports a rotatable vertical shaft 12 driven by an electric motor with conic transmission gears 16 and 18. The electric motor 14 is connected to a timer 20 which can supply electrical energy to the motor 14 for a desired length of time and which can be adjusted in time by button 22. The upper end of shaft 12 extends above the base 10 and has a support platform 24 which is rotatable and made preferably of stainless steel or other comparable material that can be sterilized at high temperatures. The support platform 24 includes a square member 26 and four extending syringe receiving chambers 28, 30, 32 and 34 branching outwardly, each chamber of which is formed with an aperture facing toward the axle 26a of the square member 26 and each chamber is spaced in relation to the aperture of the opposing chamber. The chambers 30 and 34 are positioned higher than chambers 28 and 32 relative to the axle 26a of the centrifuge. In the preferred embodiment the chambers are slightly tilted in relation to the horizontal position for about fifteen degrees to prevent the syringes inside from falling out. The four chambers 28, 30, 32 and 34 receive sterile test tubes 36 which houses each of the respective syringes. By staggering the openings of the syringe receiving chambers vertically, four loaded syringes, with pistons in full extension, can be inserted simultaneously. In the beginning of the process the shaft speed of the centrifuge is about 1000 R.p.m. and can be between 500 and 2000 R.p.m. for the longest radius of the chambers approximately 15 centimeters between the axle, the device and the bottom of the chamber. The action of the centrifuge can provide a perfectly stratified and completely pure mixture of collagen after a centrifugation of less than 20 seconds, which is a density and purity not realized by any previous procedures. After the centrifugation action, the syringes are then removed and the non-collagen materials are removed by use of another sterilized syringe needle. The remaining material is a highly purified collagen ready for injection or frozen storage in a patient collagen bank.

Because of applicant's invention described herein, implantation of autologous collagen for cosmetic surgery now becomes a practical procedure resultant from the availability of low cost quantities of autogolous collagen produced by applicant's invention.

The instant invention has been shown and described herein in what it is considered to be the most practical and preferred embodiment. It is recognized, however, that departures may be made therefrom within the scope of the invention and that obvious modifications will occur to a person skilled in the art.

## Claims

1. A method for producing collagen comprising the steps of:
collecting a sample of harvested fat tissue in a syringe;
subjecting the harvest material contained within the syringe to ultrasonic energy for a prescribed time period to destroy the fat cells therein; and
separating the harvested material after it has been subjected to ultrasonic energy with centrifugal force.

2. The method as in claim 1, wherein the ultrasonic energy is between 20 kHz and 3 (mHz).

3. The method as in claim 1, wherein:
the syringe containing the harvested fat tissue has ultrasonic energy transmitted therein to the harvested material through a probe immersed in the syringe without the piston.

4. The method as in claim 1, wherein:
ultrasonic energy is applied to the syringe by placing the syringe in a container having one or more ultrasonic transducer attached thereto; said container including a liquid for removal of heat around the syringe.

5. A device for producing collagen from harvested fat tissue comprising a syringe containing harvested fat tissue;
an ultrasonic probe which is receivable into said syringe for destroying fat tissue contained in said syringe;
power supply means for providing power to said ultrasonic means; and
centrifuge means for receiving said syringe containing said harvested material for separating collagen from said remaining destroyed fat tissues.

6. A device as in claim 5, wherein:
said centrifuge includes a plurality of separately spaced chambers mounted with chamber openings having the longitudinal axes of the chambers being set rectangularly spaced equally around the central axis of a mounting plate;
means attached to the mounting plate for rotating the chambers which receive syringes;
power supply means for rotating said mounting plate connected to said mounting plate whereby syringes containing harvested fat cells having been subjected to ultrasonic energy are subjected to centrifugal force providing separation of said human oil, and other impurities from the collagen whereby the collagen will be collected in a well separated layer.

7. A centrifuge for use in producing collagen from harvested human fat tissues or to condense and stratify the intact fat cells for grafting, comprising:
a rotatable rectangular platform
means connected to said platform for rotating said platform;
a plurality of elongated hollow tubes having a closed end and an open end, said tubes being sized to receive a syringe, said hollow end being rigidly fixed adjacent one edge of said rectangular rotatable platform such that reciprocal tubes have hollow openings facing each other but said reciprocal tubes are off center aligned so that each of said tubes can be filled with a syringe simultaneously.

8. A centrifuge as in claim 7 wherein:
said rigid platform is substantially mounted horizontally parallel to the earth and said tubes are inclined at a slight angle relative to the horizontal plane downwardly from their opening.

9. A method for producing collagen comprising the steps of:
collecting a sample of harvested fat tissue;
subjecting the harvested material to ultrasonic energy; and
separating the harvested material after it has been subjected to ultrasonic energy.

10. The method as in claim 8, wherein:
the separation is done by centrifugal force.

FIG. IA

FIG. IB

FIG. IC

FIG.2

FIG.1D

FIG.3

FIG.4